# EUROPEAN PATENT SPECIFICATION

(11) **EP 1 448 162 B1**
(45) Date of publication and mention of the grant of the patent: **26.07.2006**
(21) Application number: 02787743.0
(22) Date of filing: 19.11.2002
(51) Int. Cl.: A61K 8/41, A61K 8/44, A61K 8/365, A61Q 19/08

(54) **COMPOSITIONS COMPRISING AN ETHANOLAMINE DERIVATIVE AND ORGANIC METAL SALTS**
ZUSAMMENSETZUNGEN ENTHALTEND EIN ETHANOLAMINDERIVAT SOWIE ORGANISCHE METALLSALZE
COMPOSITIONS COMPRENANT UN DERIVE D'ETHANOLAMINE ET SELS METALLIQUES ORGANIQUES

(30) Priority: 21.11.2001 EP 01402976; 21.11.2001 EP 01402975
(43) Date of publication of application: 25.08.2004
(73) Proprietor: Johnson & Johnson Consumer France SAS, 92787 Issy les Moulineaux Cedex 9 (FR)
(72) Inventor: MORELLI, Muriel, F-27400 Acquiny (FR); LE FUR, Agnès, F-27400 Louviers (FR); ODDOS, Thierry, F-92190 Meudon (FR)
(74) Representative: Weber-Bruls, Dorothée
(86) International application number: PCT/EP2002/013009
(87) International publication number: WO 2003/043596

(56) References cited:
- EP-A- 0 029 000
- EP-A- 0 109 102
- WO-A-01/17486
- FR-A- 2 604 625
- GB-A- 474 820
- US-A- 3 205 135
- US-A- 4 938 969
- US-A- 5 643 586
- US-A- 5 847 003
- US-A- 5 879 690
- US-A- 5 922 346
- US-A- 6 162 419
- US-A1- 5 554 647

## Description

### Brief description of the invention

This invention relates to compositions comprising an ethanolamine derivative and an organic salt of zinc and/or magnesium. It further relates to the use of such compositions in topical formulations, in particular in anti-aging formulations. The invention further relates to the use of such compositions to promote human dermal fibroblasts growth and to combat the effects of skin aging.

### Background of the Invention

Skin is composed of three integrated layers: the epidermis, the dermis and the hypodermis. The thickness of the epidermis and the dermis varies over different parts of the body. The epidermis also grows into fingernails, toenails and hair. The epidermis is principally composed of three types of cells: keratinocytes (90% of epidermal cells), the melanocytes (2-8% of epidermal cells) and Langerhans' cells.

The dermis, or true skin, is thick, sturdy, rich in nerves and blood vessels and in perspiratory glands. It also functions to shield and repair injured tissue. The dermis consists mostly of collagen, which originates from cells called fibroblasts, elastin structural glycoproteins and proteoglycans. Collagen fibers, which represent 70% of the dry weight of the dermis, form a supporting mesh responsible for skin's mechanical characteristics such a strength, texture and resilience. Other cells such as macrophages and leukocytes are also present in the dermis layer.

The hypodermis, joined to the bottom of the dermis, is the deepest layer of the skin. It contains so-called 'adipocytes' which produce lipids that build the fatty layer in the subcutaneous tissue. This layer functions to protect muscles, bones and inner organs against shocks, and to act as an insulator and source of energy during lean times.

As a first sign of aging, skin becomes less elastic and develops fine lines and wrinkles, which are the direct result of deterioration of the supporting dermis layer. In fact the skin's ability to replace damaged collagen diminishes and more and more disconnections and irregularities develop in the collagen network. Further phenomena associated with skin aging are the appearance of pigment marks, skin thinning and skin sagging. Many factors contribute to accelerated collagen damage. These include sun exposure, the presence of free radicals, some age-related hormonal changes, and smoking.

Aging of the skin is attributed to two causal factors. On the one hand there is chronological or intrinsic aging while on the other there is extrinsic aging, or aging due to environmental factors. Amongst the latter there can be mentioned photo-aging, which is the damage caused to the skin due to direct or indirect effects of the ultraviolet spectrum of sunlight.

A number of treatments have been developed that have proved out to be more or less effective in combating the effects of skin aging. Cosmetic products have been introduced which contain vitamins or vitamin derivatives, in particular vitamin A or its derivatives (retinoids), vitamin C, alpha-hydroxy acids or plant extracts. These products, when applied regularly during longer periods of time, reduce the number of wrinkles and fine wrinkles. Collagen implants on the other hand can disguise expression lines around the eyes and mouth. Dermabrasion and chemical peels are applied to remove the top layer of damaged skin. Carbon dioxide laser resurfacing is applied to remove fine wrinkles and improve scars.

A particular pathway used in the treatment of the effects of skin aging is by stimulation of dermal human fibroblasts and collagen formation. Agents possessing these properties for example are L-ascorbic acid and in particular retinol.

Other agents that have been described to be useful to treat the effects of skin aging are the ethanolamine derivatives. US 5,554,647 describes a method of treating aging skin and subcutaneous muscles comprising the use of an acetylcholine precursor such as dimethylaminoethanol (DMAE) in an amount effective to produce increased muscle tone.

US 5,643,586 describes the topical treatment of subcutaneous muscle and overlying cutaneous tissue by applying a composition comprising a catecholamine precursor which in particular is tyrosine, phenylalanine or a mixture thereof preferably in combination with an acetylcholine precursor such as dimethylaminoethanol.

Metals salts of magnesium, zinc or copper have been described to possess a beneficial effect on cell metabolism and on extracellular matrix synthesis in dermal fibroblasts.

Although these methods and the products used therein have been applied with varying degrees of success, there nevertheless remains room for improvement. In particular there still is a need for new formulations that are more effective in combating the effects of the skin aging process and in particular promote skin cell renewal and extracellular matrix production. One way to achieve this goal would be to combine certain active ingredients in one formulation.

It is therefore an object of this invention to provide new compositions that contain a combination of an ethanolamine and one or more other active ingredients, that are more effective in combating the effects of skin aging. It is a further object to provide such compositions that possess an increased beneficial effect on cell metabolism and on extracellular matrix synthesis in dermal fibroblasts.

These objects are attained by the compositions and formulations according to the present invention, which will be described hereinafter in more detail.

### Summary of the invention

The present invention is directed to a composition comprising at least one ethanolamine derivative of formula (I), or a topically acceptable salt thereof: 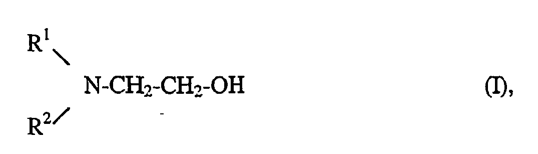 and zinc gluconate and magnesium aspartate.

In formula (I) R¹ and R² independently represent hydrogen, C₃₋₆ cycloalkyl or C₁₋₆ alkyl, optionally substituted with hydroxy, methoxy, oxo or formyl.

Preferably R¹ and R² independently represent C₁₋₄ alkyl.

The most preferred ethanolamine of formula (I) is dimethylaminoethanol (DMAE), also referred to as deanol. Preferred salts are alpha hydroxy acid salts. Most preferred salts are glycolic and citric acid salts, or combined salts.

The compositions of the invention may contain further organic salts of other metals, in particular of copper. These salts preferably are derived from the same acids as described above or hereinafter in respect to the zinc or magnesium salts or are derived from naturally occurring amino acids or from hydroxyalkyl acids.

The invention also comprises the use of a composition as specified herein to promote growth of human dermal fibroblasts.

The invention further is concerned with a topical formulation comprising a composition as defined herein and further ingredients. The topical formulation can be for dermatological use but is in particular for cosmetic use.

In another aspect the invention provides the use of a composition as defined herein for manufacturing a topical or in particular a cosmetic formulation. The topical or cosmetic formulations in particular are useful for combating or treating the effects of skin aging.

In a further aspect the invention provides the use, and in particular the cosmetic use, of a composition as defined herein, or of a topical formulation as defined herein, for combating or treating the effects associated with the aging of skin.

Or, alternatively, the invention concerns a method, and in particular a cosmetic method, of combating or treating the effects of skin aging, which method or cosmetic method comprises applying to the affected skin area an amount of a composition or a topical formulation as defined herein, said amount being effective to treat said effects of skin aging.

Still another aspect of this invention comprises a cosmetic method for the improvement of the external appearance of an individual, said method comprising applying a composition or a topical composition as defined herein to affected skin areas.

### Detailed description of the invention

The compositions of the present invention contain an ethanolamine of formula (I) as defined above. As used herein C₃₋₆ cycloalkyl refers to a cyclic cycloalkyl radical having from 3 to 6 carbon atoms and that preferably is saturated such as cyclopropyl, cyclobutyl, cyclopentyl and cyclohexyl. C₁₋₆ alkyl refers to straight and branch chained hydrocarbon radicals which preferably are saturated and have from 1 to 6 carbon atoms such as, for example, methyl, ethyl, n.propyl, iso-propyl, n.butyl, iso-butyl, t.butyl, n. pentyl, iso-pentyl, 2-methylbutyl, 2,2-dimethylpropyl, n-hexyl, 2-methylpentyl, 2,2-dimethylbutyl and the like. C₁₋₄ alkyl refers to the same group of radicals with those having 5 or 6 carbon atoms being excluded.

A particularly preferred ethanolamine of formula (I) is DMAE (deanol).

The ethanolamines of formula (I) can be prepared according to art-known procedures, e.g. by alkylating ethanolamine.

The ethanolamines of formula (I) can be used in its basic form or can be used as an appropriate topically acceptable salt, the latter referring to acid-addition salt forms that are biologically acceptable for the skin and/or mucous membranes. Biologically acceptable in particular refers to lack of toxicity and of adverse effects such as irritation, allergic reactions and the like.

Suitable topically acceptable salts are those that are obtained by treating the base form of the ethanolamine of formula I with an appropriate acid. Suitable acids for use in the preparation of the ethanolamine salts according to the invention include any inorganic or, which is preferred, organic acid known to be useful in skin care compositions. These include acids such as, for example, inorganic acids such as hydrohalic acids, e.g. hydrochloric or hydrobromic acid; boric, sulfuric, nitric, phosphoric and the like acids; or organic acids such as, for example, acetic, propanoic, hydroxyacetic (or glycolic), lactic, pyruvic, oxalic, malonic, succinic, maleic, fumaric, malic, tartaric, citric, methanesulfonic, ethanesulfonic, benzenesulfonic, p-toluenesulfonic, cyclamic, salicylic, *p*-aminosalicylic, ascorbic and the like acids.

In a preferred embodiment, at least one of the acids is an alpha hydroxy acid, such as taught for example in U.S. Patent No. 5,856,357, the disclosure of which is hereby incorporated by reference. Particularly preferred is a mixture of at least two of glycolic acid, malic acid and citric acids. In a most preferred embodiment, the acid is a combination of glycolic acid and either malic or citric acid. In situations where pH stability is a particular concern, e.g., long term storage, a particularly preferred embodiment is when the acid is a mixture of citric and glycolic acid. Preferably, the ratio of malic or citric acid to glycolic acid ranges from about 1:1 to about 1:5, more preferably, from about 1:1 to about 1:3.

Another compound which is advantageously present in the compositions of this invention is tyrosine. Tyrosine may be present in the compositions of this invention in the amount of from about 0.01 to about 5%, more preferably from about 0.04 to about 3% by weight and most preferably about 0.5% by weight, based on the total composition.

The compositions of the invention further contain at least a topically acceptable organic salt of zinc or magnesium. Topically acceptable salt forms of zinc are those that are biologically acceptable for the skin and/or mucous membranes. These salts in particular will lack irritation and show a sufficiently low level of toxicity. The acids from which these salts are derived are organic acids which will be selected such that they fulfill these requirements.

Particular organic salts of zinc or magnesium for use in the compositions of the present invention are organic acids such as, for example, acetic acid, ascorbic acid, benzoic acid, carbonic acid, citric acid, lactic acid, lauric acid, pidolic acid, propanoic acid, myristic acid, palmitic acid, salicylic acid, stearic acid, tartaric acid, malic acid and the like acids. Other organic acids are derived from naturally occurring amino acids such as, for example, aspartic acid. Other particular salts are derived from mono- or polyhydroxyalkyl carboxylic acids. As used herein mono- or polyhydroxyalkyl carbonic acids refers to carbonic acids having an alkyl chain being mono- or polysubstituted with hydroxy groups with a maximum of one hydroxy group per carbon atom, and wherein the said carbonic acids have from 2 to 7 carbon atoms. A preferred subgroup of said polysubstituted hydroxyalkyl carbonic acids are those that are derived from sugars, in particular from sugars having 5 or 6 carbon atoms, more preferably gluconic acid.

The compositions of the invention contain zinc gluconate and magnesium aspartate.

The compositions of the invention may contain other organic metal salts, in particular organic salts of copper. These other salts are derived from the same acids as the zinc salts described herein and preferably are derived from aspartic acid or gluconic acid.

In a more preferred embodiment, there is provided a composition as defined in claim 1, additionally comprising at least one topically acceptable salt of copper. Particular copper salts for use in said more preferred embodiment are the aspartate or gluconate salts.

Particularly preferred embodiments are those compositions that contain one or more salts selected from magnesium aspartate, zinc gluconate and copper gluconate. Of particular interest are compositions containing magnesium aspartate, zinc gluconate and copper gluconate.

The organic metal salts used in the compositions of the invention are commercially available or can be prepared by methods known in the art.

The w/w ratio of the magnesium and zinc salts is in the range of about 2 : 1 to 1 : 2, preferably from about 1.5 : 1 to 1 : 1.5, more preferably the w/w ratio is about 1 : 1. If present, the copper salts will be present in an amount equal or, which is preferred, lower than that of the magnesium and/or zinc salts. The w/w ratio of the copper salts to the magnesium and/or zinc salts may be in the range of about 1 : 1 to 1 : 20, preferably from about 1 : 5 to 1 : 15, more preferably said w/w ratio is about 1 : 10.

A particularly preferred organic salt mixture is the mixture containing magnesium aspartate, zinc gluconate and copper gluconate. Such a mixture is commercially available under the trade name Sepitonic M3^{™}, available from the company Seppic. Sepitonic M3^{™} is an aqueous solution containing 4.5% of magnesium aspartate, 4.5% of zinc gluconate, 0.5% of copper gluconate, water and some preservative.

In the compositions of the present invention the w/w ratio of the amount of ethanolamine (I) or the salts thereof, to the said total amount of organic metal salts may vary but in particular is in the range of about 100 : 1 to 1 : 1, further in particular from 50 : 1 to 1 : 1 or from 30 : 1 to 1 : 1, still further in particular from 20: 1 to 2 : 1, preferably from about 20 : 1 to 5 : 1, more preferably the w/w ratio is in the range of about 20 : 1 to 10 : 1. These w/w ratios relate to the total amount of dry ethanolamine (I) or the salts thereof, and the total amount of the dry metal salts. Hence if the salts are used in solution, e.g. when using the Sepitonic M3^{™} product mentioned above, the quantity of salts need to be recalculated in function of their dilution. For example in the particular case where Sepitonic M3^{™} is used, the total concentration in this product of the salts is 10% and hence for each part by weight of the metal salts that is desired in a certain formulation, a tenfold of parts by weight of Sepitonic M3^{™} product should be used.

The said organic salts preferably are formulated in an aqueous medium, optionally in the presence of water-soluble organic components such as mono- or polyalkanols, in particular polyalkanols having emollient properties such as glycerine.

The compositions of the invention are prepared by adding the aqueous formulations of the salts are to the ethanolamine component or vice versa. The compositions of the present invention therefore are mostly of aqueous nature.

This invention further relates to topical formulations containing a composition as defined herein. Topical compositions comprise as well dermatological formulations (or topical pharmaceutical formulations), and cosmetical formulations. Said topical formulations may further contain other ingredients or additives used in dermatological or in cosmetical formulations, including other active ingredients. Examples of further ingredients or additives are surfactants, emulsifiers, consistency factors, conditioners, emollients, skin caring ingredients, moisturizers, thickeners, lubricants, fillers, binding agents, anti-oxidants, preservatives, active ingredients, in particular dermatologically active ingredients, fragrances and the like. Active ingredients as mentioned herein comprise, for example, anti-inflammatories, anti-bacterials, anti-fungals and the like agents. Of particular interest are any active ingredients suited for topical applications.

The topical formulations according to the invention can further include one or more of a variety of optional ingredients, such as coloring agents, opacifying agents and the like.

The ethanolamine and more specifically dimehylethanolamine should be used in the formulations for topical application at concentrations from 0.001% to 10% and preferably from 0.1% to 5%. The organic salts of metals taken together should be used in the formulations for topical application between 0.001% to 1% and preferably from 0.01 to 0.2%.

Unless indicated otherwise, all percentages in the preceding and following paragraphs are w/w percentages.

The formulations according to the present invention can be prepared by adding the appropriate ingredients to a composition of the invention, or vice versa by adding the composition to an appropriate cosmetic or dermatological formulation base. It is also possible to mix all the ingredients individually, i.e. without making a separate composition as defined herein.

The compositions and topical formulations subject of the present invention are useful to combat or to treat the effects of skin aging. The effects of skin aging comprise those associated with the aging of the skin such as the appearance of fine lines, fine wrinkling, wrinkling, loss of skin firmness, skin tightening and suppleness. The effects of skin aging are due to aging as such, but also to aging of the skin caused by external factors such as exposure to environmental factors such as exposure to sunlight, wind, atmospheric pollutants and the like, or a combination of these factors.

Certain ethanolamine derivatives and in particular DMAE are known to stimulate the proliferation of dermal cells and in particular the proliferation of fibroblasts. This is also the case with certain organic salts of copper, zinc and magnesium. It now has been found the ethanolamine analogs of formula (1), and in particular DMAE, and the organic salts of zinc or of magnesium as specified herein, act synergistically. For example these combinations show synergistic effects in their stimulation of the proliferation of dermal cells and in particular the proliferation of fibroblasts. Similar synergistic effects are present in mixtures containing the ethanolamine analogs of formula (I), and in particular DMAE, as well as the organic salts of zinc or magnesium as specified herein. Additionally, synergistic effects are present in mixtures containing the ethanolamine analogs of formula (I), and in particular DMAE, as well as the organic salts of magnesium and of copper as specified herein. Mixtures of ethanolamines of formula (I) and organic salts of zinc, magnesium and copper additionally show effects on collagen production (stimulation of collagen synthesis). This is especially the case in the instance where use is made of a mixture of an ethanolamine (I) and zinc gluconate, magnesium aspartate and copper gluconate.

The topical formulations according to the invention may be in the form of a solution, a hydrophilic lotion, an ointment, a cream or a gel. The formulations may also be, for example, in the form of oil-in-water, water-in-oil or multiple emulsions, foaming products or in liposome form.

Preferred formulations are gel and cream based formulations. Of particular interest are formulations based on oil-in-water emulsions.

In the latter instance an oily phase, containing the oil-soluble components, is made separately which is added to any of the aqueous phases containing suitable emulsifiers. Preferably the first aqueous phase is made containing one or more suitable emulsifiers. The oily phase is made and added to the first phase while building an emulsion. Subsequently the second aqueous phase is added. In the instance where the first or oily phase contains solid or semi-solid components, it is recommendable to heat the phase or phases and to conduct the emulsifying process at elevated temperature.

The topical formulations according to the invention can further include one or more of a variety of additional ingredients commonly found in skin care compositions, such as for example, emollients, skin conditioning agents, emulsifying agents, humectants, preservatives, antioxidants, active ingredients, perfumes, chelating agents, dyes, opacifying agents, etc., provided that they are physically and chemically compatible with the other components of the composition. Notably useful is the incorporation of vitamin A and vitamin A derivatives, including but not restricted to retinol, retinyl palmitate, retinoic acid, retinal, and retinyl propionate.

Examples of other active agents which may be incorporated comprise anti-microbials, e.g. anti-bacterials and antifungals, anti-inflammatory agents, anti-irritating compounds, anti-itching agents, moisturising agents, skin caring ingredients, plant extracts, vitamins, and the like. Also included are sunscreen actives which may be inorganic or organic in nature.

Examples of suitable preservatives for use in the compositions or formulations of the invention include the C₁-C₄ alkyl parabens and phenoxyethanol. Generally, the preservative is present in an amount ranging from about 0.5 to about 2.0, preferably about 1.0 to about 1.5, weight percent based on the total composition. In a preferred embodiment, the preservative is mixture of from about 0.2 to about 0.5 weight percent methylparaben, from about 0.2 to about 5.0 weight percent propylparaben and from about 0.05 to about 0.10 weight percent butylparaben. A particularly preferred commercially available preservative that may be used in the skin care composition according to this invention is Phenonip^{™} which is a practically colorless, viscous, liquid mixture of phenoxyethanol, methylparaben, ethylparaben, propylparaben, and butylparaben available from Nipa Laboratories, Inc.

Preferably, antioxidants should be present in the compositions or formulations according to the invention. Suitable antioxidants include butylated hydroxy toluene (BHT), ascorbyl palmitate, butylated hydroanisole (BHA), phenyl-α-naphthylamine, hydroquinone, propyl gallate, nordihydroquiaretic acid, vitamin E or derivatives of vitamin E, vitamin C and derivatives thereof, calcium pantothenic, green tea extracts and mixed polyphenosls, and mixtures thereof of the above. When utilized the antioxidant can be present in an amount ranging from about 0.02 to about 0.5% by weight, more preferably from about 0.002 to about 0.1% by weight of the total composition.

Emollients which can be included in the compositions or formulations of the invention function by their ability to remain on the skin surface or in the stratum corneum to act as lubricants, to reduce flaking, and to improve the skin appearance. Typical emollients include fatty esters, fatty alcohols, mineral oil, polyether siloxane copolymers and the like. Examples of suitable emollients include, but are not limited to, polypropylene glycol ("PPG")-15 stearyl ether, PPG-10 cetyl ether, steareth-10, oleth-8, PPG-4 lauryl ether, vitamin E acetate, PEG-7 glyceryl cocoate, lanolin, cetyl alcohol, octyl hydroxystearate, dimethicone, and combinations thereof. Cetyl alcohol, octyl hydroxystearate, dimethicone, and combinations thereof are preferred. When utilized, the emollient can be present in an amount from about 0.01 to about 5, preferably from about 1 to about 4 percent by weight based on the total composition.

Polyhydric alcohols can be utilized as humectants in the compositions or formulations of the invention. The humectants aid in increasing the effectiveness of the emollient, reduce scaling, stimulate removal of built-up scale and improve skin feel. Suitable polyhydric alcohols include, but are not limited to, glycerol (also known as glycerin), polyalkylene glycols, alkylene polyols and their derivatives, including butylene glycol, propylene glycol, dipropylene glycol, polypropylene glycol, polyethylene glycol and derivatives thereof, sorbitol, hydroxypropyl sorbitol, hexylene glycol, 1,3-dibutylene glycol, 1,2,6,-hexanetriol, ethoxylated glycerol, propoxylated glycerol and mixtures thereof. Glycerin is preferred. When utilized, the humectant is present in an amount from about 0.1 to about 5, preferably from about 1 to about 3 percent by weight, based on the total weight of the composition.

The topical formulations of the invention have an improved effect on skin damage due to aging. The first results may be obtained after four weeks of treatment with the compositions and are exerted deep down. These effects comprise a reduction in the number and depth of wrinkles and small wrinkles, a firming and tightening of the skin and providing a more youthful and smooth aspect of the skin, in particular of facial skin.

All the topical formulations as described above can be applied on the skin by means of wipes.

The topical formulations of the invention may be applied in the morning and/or evening. They may be applied on those parts of the body where skin aging is prominent, i.e. on the face, the body or the hands.

The following examples are meant to illustrate the invention and not to limit it thereto.

### Examples

### Example 1

### Formulations

In the following formulation examples all percentages are by weight (w/w).

The formulations were prepared in the following manner. First an aqueous phase was made which was heated. Subsequently the gellyfying agents were added. The oily components were melted and mixed into an oily phase which was emulsified in the water phase
The thus formed emulsion was allowed to cool and the active ingredients were added at a temperature of 35°C. Then the fragrance was added at a temperature of 30°C.

**Formula 1**

| Components | % |
|---|---|
| Aqua | 63.794 |
| Acrylate / C10/30 alkyl acrylates crosspolymer | 0.35 |
| Cetyl phosphate | 1.88 |
| Aqua, Glyceryl polymethacrylate, propylene glycol | 5 |
| Phenoxyethanol | 0.73 |
| Methylparaben | 0.2 |
| Propylparaben | 0.07 |
| Cetearyl octanoate, isopropyl myristate | 8 |
| Cetyl palmitate | 4 |
| Cetyl alcohol | 1.5 |
| Dimethicone | 0.5 |
| Polyglyceryl 3 diisostereate | 1 |
| Tocopheryl acetate | 1 |
| Cetearyl isononanoate | 6 |
| Dimethyl AE | 1 |
| Aqua | 3 |
| Citric Acid | 0.096 |
| Aqua, Glycolic Acid | 0.13 |
| Aqua/ Zinc gluconate/ glycerine 50%/5%/45% | 1 |
| Sodium carboxymethyl betaglucan | 0.5 |
| Perfume | 0.25 |

**Formula 2**

| Components | % |
|---|---|
| Aqua | 63.794 |
| Acrylate / C10/30 alkyl acrylates crosspolymer | 0.35 |
| Cetyl phosphate | 1.88 |
| Aqua, Glyceryl polymethacrylate, propylene glycol | 5 |
| Phenoxyethanol | 0.73 |
| Methylparaben | 0.2 |
| Propylparaben | 0.07 |
| Cetearyl octanoate, isopropyl myristate | 8 |
| Cetyl palmitate | 4 |
| Cetyl alcohol | 1.5 |
| Dimethicone | 0.5 |
| Polyglyceryl 3 diisostereate | 1 |
| Tocopheryl acetate | 1 |
| Cetearyl isononanoate | 6 |
| Dimethyl AE | 1 |
| Aqua | 3 |
| Citric Acid | 0.096 |
| Aqua, Glycolic Acid | 0.13 |
| Aqua/ Magnesium aspartate/ Zinc gluconate/ | 1 |
| glycerine 45%/5%/5%/45% | |
| Sodium carboxymethyl betaglucan | 0.5 |
| Perfume | 0.25 |

**Formula 3**

| Components | % |
|---|---|
| Aqua | 63.794 |
| Acrylate / C10/30 alkyl acrylates crosspolymer | 0.35 |
| Cetyl phosphate | 1.88 |
| Aqua, Glyceryl polymethacrylate, propylene glycol | 5 |
| Phenoxyethanol | 0.73 |
| Methylparaben | 0.2 |
| Propylparaben | 0.07 |
| Cetearyl octanoate, isopropyl myristate | 8 |
| Cetyl palmitate | 4 |
| Cetyl alcohol | 1.5 |
| Dimethicone | 0.5 |
| Polyglyceryl 3 diisostereate | 1 |
| Tocopheryl acetate | 1 |
| Cetearyl isononanoate | 6 |
| Dimethyl AE | 1 |
| Aqua | 3 |
| Citric Acid | 0.096 |
| Aqua, Glycolic Acid | 0.13 |
| Aqua/ Magnesium aspartate/ Zinc gluconate/ | 1 |
| glycerine 45%/5%/5%/45% | |
| Sodium carboxymethyl betaglucan | 0.5 |
| Perfume | 0.25 |

**Formula 4**

| Components | % |
|---|---|
| Aqua | 63.794 |
| Acrylate / C10/30 alkyl acrylates crosspolymer | 0.35 |
| Cetyl phosphate | 1.88 |
| Aqua, Glyceryl polymethacrylate, propylene glycol | 5 |
| Phenoxyethanol | 0.73 |
| Methylparaben | 0.2 |
| Propylparaben | 0.07 |
| Cetearyl octanoate, isopropyl myristate | 8 |
| Cetyl palmitate | 4 |
| Cetyl alcohol | 1.5 |
| Dimethicone | 0.5 |
| Polyglyceryl 3 diisostereate | 1 |
| Tocopheryl acetate | 1 |
| Cetearyl isononanoate | 6 |
| Dimethyl AE | 1 |
| Aqua | 3 |
| Citric Acid | 0.096 |
| Aqua, Glycolic Acid | 0.13 |
| Sepitonic M3™ | 1 |
| Sodium carboxymethyl betaglucan | 0.5 |
| Perfume | 0.25 |

### Example 2

### Test

Fibroblasts were prepared from an abdominal biopsy of a 66 old female patient undergoing plastic surgery. Fibroblasts were seeded in 24 multiwell plates at 25,000 cells per well in 1 ml of DMEM supplemented with fetal calf serum (FCS) and antibiotics. Cultures were incubated 24 hours at 37° C to allow cell attachment and spreading. Supernatants were then eliminated and replaced with fresh medium supplemented with 2% of FCS and containing the tested product at different concentrations. After 10 days of culture, cells were trypsinized and counted with a Coulter counter.

### Results

Effect of DMAE, Zinc gluconate and their association on fibroblasts proliferation after 10 days of culture :

| | Mean of cell/ml | % effect vs control |
|---|---|---|
| Control | 76900 ± 1990 | |
| DMAE 10⁻⁴ M | 100 727 ± 1963^{*} | 31 |
| DMAE 10⁻⁵ M | 88 013 ± 2733^{*} | 14 |
| Zn gluconate 500 ng/ml | 85 647 ± 1760^{*} | 11 |
| Zn gluconate 50 ng/ml | 79 367 ± 1059 | 3 |
| Zn gluconate 5 ng/ml | 76 773 ± 4806 | 0 |
| DMAE 10⁻⁴ M | ± | |
| + Zn gluconate 500 ng/ml | 105 813 ± 4286^{*¤} | 38 |
| + Zn gluconate 50 ng/ml | 101 187 ± 4614^{*¤} | 32 |
| + Zn gluconate 5 ng/ml | 112 720 ± 4660^{*§¤} | 47 |
| DMAE 10⁻⁵ M | | |
| + Zn gluconate 500 ng/ml | 97533 ± 9185^{*} | 27 |
| + Zn gluconate 50 ng/ml | 92 200 ± 2864^{*¤} | 20 |
| + Zn gluconate 5 ng/ml | 104 047 ± 3057^{*§¤} | 35 |

| | | |
|---|---|---|
| * Significant difference versus untreated control with p < 0.05 | | |
| § Significant difference versus DMAE with p < 0.05 | | |
| ¤ Significant difference versus Zinc gluconate with p < 0.05 | | |

Effect of DMAE, Magnesium aspartate and their association on fibroblasts proliferation after 10 days of culture :

| | **Mean of cell/ml** | **% effect vs control** |
|---|---|---|
| Control | 76900 ± 1990 | |
| DMAE 10⁻⁴ M | 100727 ± 1963^{*} | 31 |
| DMAE 10⁻⁵ M | 88 013 ± 2733^{*} | 14 |
| Mg aspartate 500 ng/ml | 92753 ± 2771^{*} | 21 |
| Mg aspartate 50 ng/ml | 79400 ± 1674 | 3 |
| Mg aspartate 5 ng/ml | 78073 ± 4231 | 2 |
| DMAE 10⁻⁴ M | | |
| + Mg aspartate 500 ng/ml | 97267 ± 4561^{*} | 26 |
| + Mg aspartate 50 ng/ml | 86773 ± 5375^{*§¤} | 13 |
| + Mg aspartate 5 ng/ml | 104727 ± 3133^{*¤} | 36 |
| DMAE 10-⁵ M | | |
| + Mg aspartate 500 ng/ml | 92827 ± 4580^{*} | 21 |
| + Mg aspartate 50 ng/ml | 77667 ± 2443^{§} | 1 |
| + Mg aspartate 5 ng/ml | 99660 ± 4924^{*§¤} | 30 |

| | | |
|---|---|---|
| * Significant difference versus untreated control with p < 0.05 | | |
| § Significant difference versus DMAE with p < 0.05 | | |
| ¤ Significant difference versus Mg aspartate with p < 0.05 | | |

## Claims

1. Composition comprising at least one ethanolamine derivative of formula (I), or a topically acceptable salt thereof: R¹R²NCH₂CH₂OH (I), and zinc gluconate and magnesium aspartate, wherein in formula (I) R¹ and R² independently represent hydrogen, C₃₋₆ cycloalkyl or C₁₋₆ alkyl, optionally substituted with hydroxyl, methoxy, oxo or formyl.

2. The composition according to claim 1, further containing organic salts of copper.

3. The composition according to claim 2, wherein the organic salt of copper is copper gluconate.

4. The composition according to claims 1 to 3, wherein R¹ and R² independently represent C₁₋₄ alkyl.

5. The composition according to claims 1 to 4, wherein the ethanolamine derivative of formula (I) is dimethylaminoethanol (DMAE).

6. The composition according to claims 1 to 5, wherein the topically acceptable salt of the ethanolamine derivative of formula (I) is a glycolic or citric acid salt.

7. A topical formulation comprising a composition as claimed in any of claims 1 to 6 and further ingredients.

8. The topical formulation according to claim 7, wherein the further ingredient is selected from the group consisting of surfactants, emulsifiers, consistency factors, conditioners, emollients, skin caring ingredients, moisturizers, thickeners, lubricants, fillers, binding agens, anti-oxidants, preservatives, anti-inflammatory agents, anti-bacterial agents, anti-fungal agents, and mixtures thereof.

9. Use of a composition as claimed in claims 1 to 6 for manufacturing a topical or in particular a cosmetic formulation.

## Patentansprüche

1. Zusammensetzung, umfassend zumindest ein Ethanolaminderivat der Formel (I) oder ein topisch akzeptables Salz desselben: R¹R²NCH₂CH₂OH (I) und Zinkgluconat und Magnesiumaspartat, wobei in Formel (I) R¹ und R² unabhängig voneinander Wasserstoff, C₃₋₆-Cycloalkyl oder C₁₋₆-Alkyl darstellen, optional substituiert mit Hydroxyl, Methoxy, Oxo oder Formyl.

2. Die Zusammensetzung nach Anspruch 1, weiterhin umfassend organische Salze des Kupfers.

3. Die Zusammensetzung nach Anspruch 2, **dadurch gekennzeichnet, daß** das organische Salz des Kupfers Kupfergluconat ist.

4. Die Zusammensetzung nach Ansprüchen 1 bis 3, **dadurch gekennzeichnet, daß** R¹ und R² unabhängig voneinander C₁₋₄-Alkyl darstellen.

5. Die Zusammensetzung nach Ansprüchen 1 bis 4, **dadurch gekennzeichnet, daß** das Ethanolaminderivat der Formel (I) Dimethylaminoethanol (DMAE) ist.

6. Die Zusammensetzung nach Ansprüchen 1 bis 5, **dadurch gekennzeichnet, daß** das topisch akzeptable Salz des Ethanolaminderivats der Formel (I) ein Glykol- oder Citronensäuresalz ist.

7. Topische Formulierung, umfassend eine Zusammensetzung wie in einem der Ansprüche 1 bis 6 beansprucht und weitere Inhaltsstoffe.

8. Die topische Formulierung nach Anspruch 7, **dadurch gekennzeichnet, daß** der weitere Inhaltsstoff aus der Gruppe ausgewählt wird, die sich aus grenzflächenaktiven Stoffen, Emulgatoren, Konsistenzfaktoren, Zusatzstoffen, Weichmachern, Hautpflegestoffen, Feuchthaltestoffen, Eindickem, Schmiermitteln, Füllstoffen, Bindemitteln, Antioxidationsmitteln, Konservierungsstoffen, entzündungshemmenden Stoffen, antibakteriellen Stoffen, antifungalen Stoffen und Gemischen daraus zusammensetzt.

9. Verwendung einer Zusammensetzung wie in Ansprüchen 1 bis 6 beansprucht zur Herstellung einer topischen oder insbesondere einer kosmetischen Formulierung.

## Revendications

1. Composition comprenant au moins un dérivé d'éthanolamine de formule (I), ou un sel acceptable d'un point de vue topique de celui-ci : R¹R²NCH₂CH₂OH (I), et du gluconate de zinc et de l'aspartate de magnésium, dans laquelle dans la formule (I) R¹ et R² représentent indépendamment l'hydrogène, un cycloalkyle en C₃-C₆ ou un alkyle en C₁-C₆, facultativement substitués par un hydroxyle, un méthoxy, un oxo ou un formyle.

2. Composition selon la revendication 1, contenant en outre des sels organiques de cuivre.

3. Composition selon la revendication 2, dans laquelle le sel organique de cuivre est le gluconate de cuivre.

4. Composition selon les revendications 1 à 3, dans laquelle R¹ et R² représentent indépendamment un alkyle en C₁-C₄.

5. Composition selon les revendications 1 à 4, dans laquelle le dérivé d'éthanolamine de formule (I) est le diméthylaminoéthanol (DMAE).

6. Composition selon les revendications 1 à 5, dans laquelle le sel acceptable d'un point de vue topique du dérivé d'éthanolamine de formule (I) est un sel d'acide glycolique ou citrique.

7. Formulation topique comprenant une composition selon l'une quelconque des revendications 1 à 6 et d'autres ingrédients.

8. Formulation topique selon la revendication 7, dans laquelle les autres ingrédients sont choisis dans le groupe consistant en les agents tensioactifs, les émulsifiants, les facteurs de consistance, les conditionneurs, les émollients, les ingrédients de soin de la peau, les hydratants, les épaississants, les lubrifiants, les charges, les agents de liaison, les antioxydants, les conservateurs, les agents anti-inflammatoires, les agents antibactériens, les agents antifongiques, et des mélanges de ceux-ci.

9. Utilisation d'une composition selon l'une des revendications 1 à 6 pour la fabrication d'une formulation topique ou en particulier cosmétique.
